(19) Europäisches Patentamt
European Patent Office
Office européen des brevets

(11) Veröffentlichungsnummer : **0 231 149**
**B1**

(12) # EUROPÄISCHE PATENTSCHRIFT

(45) Veröffentlichungstag der Patentschrift : **19.10.88**

(51) Int. Cl.⁴ : **C 07 C149/273**, C 08 K 5/37

(21) Anmeldenummer : **87810033.8**

(22) Anmeldetag : **19.01.87**

(54) **Mercaptan enthaltende Polyphenole.**

(30) Priorität : **23.01.86 CH 253/86**

(43) Veröffentlichungstag der Anmeldung : **05.08.87 Patentblatt 87/32**

(45) Bekanntmachung des Hinweises auf die Patenterteilung : **19.10.88 Patentblatt 88/42**

(84) Benannte Vertragsstaaten : **AT CH DE FR GB IT LI NL**

(56) Entgegenhaltungen :
**CH-A- 472 391**
**FR-A- 2 284 593**

(73) Patentinhaber : **CIBA-GEIGY AG**
**Klybeckstrasse 141**
**CH-4002 Basel (CH)**

(72) Erfinder : **Zahir, Sheik Abdul-Cader Dr.**
**Elsternstrasse 12**
**CH-4104 Oberwil (CH)**
Erfinder : **Mechera, Karl, Dr.**
**Im Baumgarten 14**
**CH-4132 Muttenz (CH)**

**EP 0 231 149 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Verbindungen mit mindestens zwei Mercaptanresten und phenolischen Hydroxylgruppen, Zusammensetzungen enthaltend ein härtbares Epoxidharz und diese Verbindungen, die daraus erhältlichen gehärteten Produkte sowie die Verwendung der härtbaren Zusammensetzungen als Klebstoffe.

Epoxidharze sind wohlbekannt und werden mit den unterschiedlichsten Härtern zur Bildung gehärteter Produkte umgesetzt.

Bismercaptanhärter sind bekannt. Diese Verbindungen sind in der Regel bereits bei Raumtemperatur aktiv. Für eine Reihe von Anwendungen ist es erwünscht, eine temperaturgesteuerte Härtungsreaktion ablaufen zu lassen. Dies lässt sich erfindungsgemäss dadurch erreichen, dass man einen Mercaptanhärter mit einer zweiten, weniger reaktiven phenolischen Hydroxylgruppe im Molekül einsetzt.

Das gehärtete Produkt haftet besonders gut auf Metallen und zeigt eine gute Beständigkeit gegen Feuchtigkeit.

Mercaptopropylphenole sind aus der US-PS 3 336 393 als Pestizide bekannt. Eine Verwendung als Härter für Epoxidharze ist dort nicht erwähnt. Ausserdem handelt es sich bei den vorbekannten Verbindungen um einkernige Monophenole.

Die vorliegende Erfindung betrifft Verbindungen der Formeln I oder II

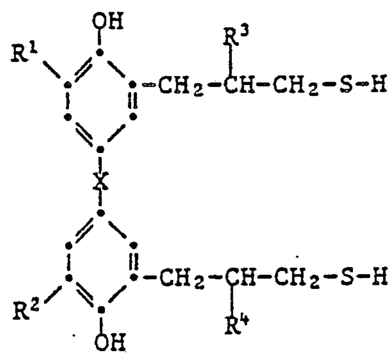

(I)

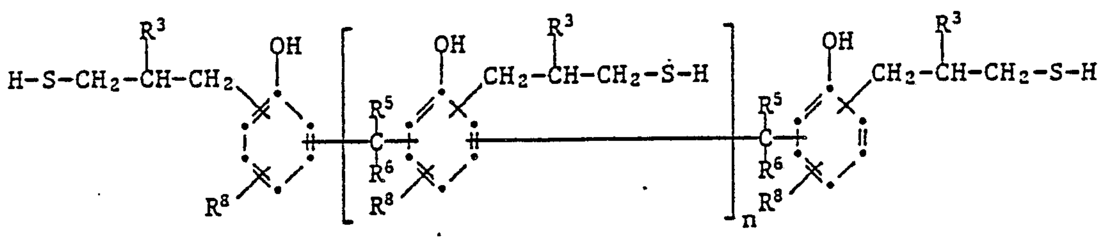

(II)

worin $R^1$ und $R^2$ unabhängig voneinander Wasserstoff, $C_1$-$C_{18}$Alkyl, $C_3$-$C_{18}$Alkenylmethyl, Cyclohexyl, Phenyl, Benzyl oder Tolyl bedeuten oder ein Rest $-CH_2-CHR^3-CH_2-S-H$ sind, $R^3$ und $R^4$ unabhängig voneinander Wasserstoff oder Methyl sind, X $-CR^5R^6-$, $-S-$, $-SO-$, $-SO_2-$ oder $-(CH_3)C[-(CH_2)_m-COOR^7]-$ bedeutet, $R^5$ und $R^6$ unabhängig voneinander Wasserstoff oder $C_1$-$C_6$Alkyl sind, $R^7$ $C_1$-$C_{18}$Alkyl ist, $R^8$ Wasserstoff, $C_1$-$C_{18}$Alkyl, $C_3$-$C_{18}$Alkenylmethyl oder Phenyl darstellt, m 1 oder 2 bedeutet und n eine ganze Zahl von 1 bis 10 ist.

$R^1$, $R^2$, $R^7$ und $R^8$ sind als $C_1$-$C_{18}$Alkyl geradkettig oder verzweigt, bevorzugt geradkettig. Bei diesen Resten handelt es sich beispielsweise um Methyl, Ethyl, Propyl, Isopropyl, n-Butyl, sek.Butyl, tert.Butyl, n-Pentyl, n-Hexyl, n-Heptyl, n-Octyl, n-Nonyl, n-Decyl, n-Dodecyl, n-Tetradecyl, n-Hexadecyl oder n-Octadecyl, sowie um 1,1,3,3-Tetramethylbutyl oder 2-Ethylhexyl.

Bevorzugt werden kurze, geradkettige Alkylreste mit 1 bis 6 Kohlenstoffatomen und ganz besonders Methyl.

Als $C_1$-$C_6$Alkyl bedeuten $R_5$ und $R^6$ beispielsweise Methyl, Ethyl, n-Propyl, n-Butyl, n-Pentyl oder n-Hexyl. Bevorzugt wird Methyl.

$C_3$-$C_{18}$Alkenylmethylreste $R^1$, $R^2$ und $R^8$ sind geradkettig oder verzweigt, vorzugsweise geradkettig. Beispiele dafür sind Allyl, But-3-enyl, Pent-4-enyl, Hex-5-enyl, Oct-7-enyl, Dec-9-enyl, Dodec-11-enyl oder Octadec-17-enyl. Bevorzugt wird Allyl.

Bevorzugt werden Verbindungen der Formel I oder II, worin $R^3$ und $R^4$ Wasserstoff bedeuten.

Ganz besonderes Interesse finden Verbindungen der Formel I, worin $R^1$ und $R^2$ unabhängig

voneinander Wasserstoff oder $C_1$-$C_{12}$Alkyl sind, $R^3$ und $R^4$ Wasserstoff bedeuten, X eine Gruppe $—CR^5R^6—$ darstellt und worin $R^5$ und $R^6$ unabhängig voneinander Wasserstoff oder Methyl sind.

Ebenfalls von Interesse sind Verbindungen der Formel I, worin $R^1$, $R^2$, $R^3$ und $R^4$ Wasserstoff sind und X eine Gruppe $—CH_2—$, $—CH(CH_3)—$ oder $—C(CH_3)_2—$ darstellt.

Ganz besonders bevorzugt werden Verbindungen der Formel I, worin $R^1$ und $R^2$ eine Gruppe $—CH_2—CHR^3—CH_2—S—H$ darstellen.

Beachtung finden ferner Verbindungen der Formel II, worin $R^3$ Wasserstoff ist, $R^8$ Wasserstoff oder Methyl bedeutet, $—CR^5R^6—$ eine Gruppe $—CH_2—$, $—CH(CH_3)—$ oder $—C(CH_3)_2—$ darstellt und worin n eine ganze Zahl von 1 bis 4 ist.

Die Gruppen $—CH_2—CHR^3—CH_2—S—H$ im Novolak der Formel II befinden sich bevorzugt in ortho- oder para-Position zur phenolischen Hydroxylgruppe ; ganz besonders bevorzugt jedoch in ortho-Position.

$R^1$ und $R^2$ sind vorzugsweise Wasserstoff oder auch Methyl, ganz besonders jedoch Wasserstoff.

$R^8$ ist bevorzugt Wasserstoff.

X ist bevorzugt $—CH_2—$ oder $—C(CH_3)_2—$, ganz besonders jedoch $—C(CH_3)_2—$.

Weiterhin bevorzugt als Gruppe X werden $—S—$ oder $—SO_2—$ oder $—(CH_3)C[—CH_2—COOCH_3]—$ oder $—(CH_3)C[—CH_2—CH_2—COOCH_3]—$.

Die Verbindungen der Formeln I oder II können beispielsweise dadurch hergestellt werden, indem man Verbindungen der Formeln III oder IV

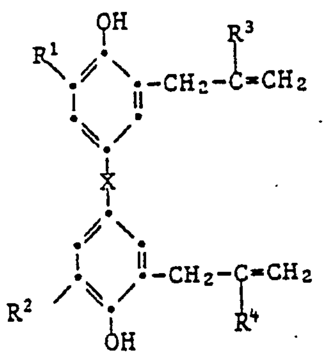

(III)

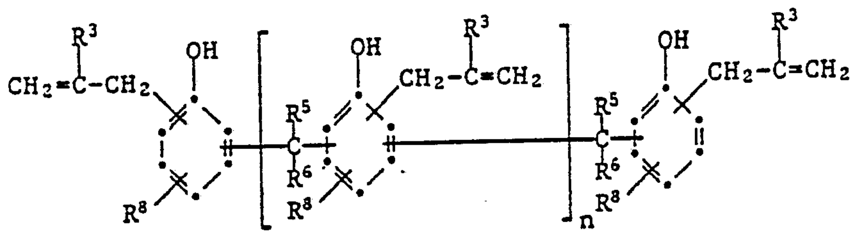

(IV)

mit einem dem Gehalt an allylständigen Doppelbindungen im wesentlichen entsprechenden molaren Anteil an Thiocarbonsäuren der Formel V

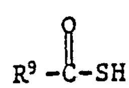

(V)

in Gegenwart eines Radikalbildners umsetzt und die Umsetzungsprodukte anschliessend verseift ; dabei besitzen die Reste $R^1$, $R^2$, $R^3$, $R^4$, $R^5$, $R^6$, $R^8$ und X, sowie der Index n die weiter oben definierte Bedeutung und $R^9$ ist ein einwertiger organischer Rest, beispielsweise Alkyl, bevorzugt Methyl ; $R^1$ und $R^2$ besitzen allerdings anstelle der Bedeutung $—CH_2—CHR^3—CH_2—S—H$ in diesem Falle die Bedeutung $—CH_2—CR^3{=}CH_2$.

Sollen Verbindungen der Formel I oder II mit Alkenylmethylresten $R^1$, $R^2$ oder $R^8$ hergestellt werden, so setzt man die Verbindungen der Formel III oder IV mit einem entsprechenden Unterschuss an Thiocarbonsäuren der Formel V um.

Der Novolak IV kann gegebenenfalls auch geringe Anteile nichtallylhaltiger Phenolkerne enthalten, beispielsweise Phenol-, o-Kresol- oder p-Kresolkerne.

Die Umsetzungsprodukte (Acylthiopropylderivate) der Formeln VI und VII

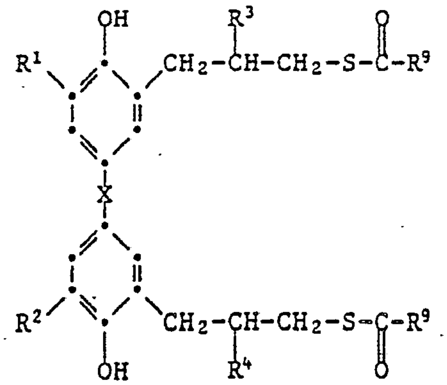

(VI)

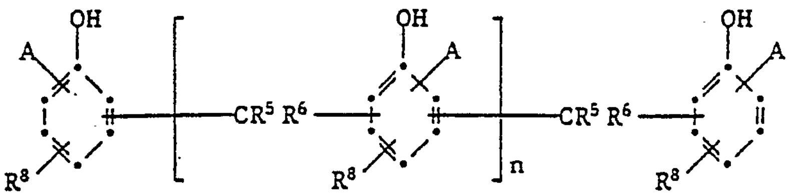

(VII)

$A = -CH_2-CHR^3-CH_2-S-\overset{O}{\overset{\|}{C}}-R^9$

werden in einer anschliessenden Hydrolysestufe in an sich bekannter Weise in die Mercaptophenole der Formeln I und II übergeführt.

Die Hydrolyse wird zweckmässig unter alkalischen Bedingungen, beispielsweise unter Einwirkung von wässriger KOH in an sich bekannter Weise durchgeführt. Die Herstellung der Verbindungen I und II kann aber auch unter Umgehung der Hydrolysestufe erfolgen, indem man die Verbindungen III oder IV direkt mit Schwefelwasserstoff in Gegenwart eines Radikalbildners umsetzt. Beide Varianten von Umsetzungen dieser Art sind in der US-PS 3 336 393 beschrieben.

Die Bis- oder Polyallylphenole bzw. -methallylphenole der Formeln III oder IV sind bekannte Verbindungen oder können nach an sich bekannten Verfahren hergestellt werden, beispielsweise durch Veretherung der entsprechenden Polyphenole mit Allylhalogenid und anschliessender Claisen Umlagerung.

Die Thiocarbonsäuren der Formel V sind ebenfalls bekannte Verbindungen und können ebenso nach an sich bekannten Verfahren erhalten werden. Vorzugsweise setzt man ein entsprechendes Carbonsäureanhydrid, bevorzugt Essigsäureanhydrid, mit Schwefelwasserstoff in alkalischer wässriger Lösung um, isoliert das entstandene Gemisch aus Carbonsäure und Thiocarbonsäure und verwendet dieses ohne weitere Auftrennung in der nachfolgenden Umsetzung mit dem (Meth)allylphenol.

Die Menge der einzusetzenden Thiocarbonsäure V oder des Schwefelwasserstoffs richtet sich nach der Anzahl der Allyl- oder Methallylgruppen im Ausgangsmaterial III oder IV. In der Regel setzt man äquimolare Mengen Thiocarbonsäure oder Schwefelwasserstoff ein, bezogen auf die Allylgruppen. Es kann aber durchaus ein Ueberschuss oder auch ein Unterschuss an Thiocarbonsäure verwendet werden.

Verwendet man einen Unterschuss an Thiocarbonsäure V, so erfolgt nur eine teilweise Umsetzung der Allylgruppen der Verbindungen III oder IV. Solche teilweise thioacylierten Produkte, insbesondere teilweise umgesetzte Novolake IV, sind ebenfalls als Zwischenprodukte zur Herstellung der erfindungsgemässen Mercaptophenole geeignet. Die entstehenden Endprodukte fallen auch unter den Rahmen der vorliegenden Erfindung. Solche teilweise umgesetzten Novolake IV sind Gemische von Verbindungen unterschiedlicher Kettenlänge und unterschiedlichen Gehaltes an Mercaptogruppen. Im Mittel sollten mindestens 50% der Allylgruppen der Verbindungen III und IV umgesetzt sein.

Die Umsetzung zum Endprodukt I oder II oder zum Zwischenprodukt VI oder VII erfolgt über einen radikalischen Mechanismus. Dazu bestrahlt man beispielsweise das Reaktionsgemisch gegebenenfalls in Anwesenheit eines Katalysators mit kurzwelligem Licht oder man erwärmt das Gemisch, vorzugsweise in Gegenwart eines Radikalbildners.

Als Radikalbildner eigenen sich beispielsweise organische Peroxide, wie Benzoylperoxid, Acetylperoxid oder Cumylhydroperoxid, und insbesondere Azoverbindungen. Von diesen wiederum sind insbesondere solche bevorzugt, bei denen die Azogruppe beidseitig an tertiäre C-Atome gebunden ist, die neben Alkylgruppen noch Nitril- oder Estergruppen tragen. So ist beispielsweise $\alpha,\alpha$-Azoisobuttersäuredinitril (AIBN) ein wichtiger Vertreter dieser Verbindungsklasse.

Bei der photoinitiierten Reaktion sind als gegebenenfalls einsetzbare Katalysatoren z. B. Benzoinether, Benzilketale, $\omega$-Dialkoxy-acetophenon-Derivate oder aromatische Keton-Amin-Kombinationen geeignet.

Die Menge an gegebenenfalls einsetzbarem Radikalbildner ist nicht kritisch und kann in weiten Grenzen schwanken ; sie beträgt vorzugsweise weniger als 10 Mol.% der Allyl- oder Methallylgruppen im Reaktionsgemisch.

Die Umsetzung der Verbindungen III und V oder IV und V bzw. III oder IV mit Schwefelwasserstoff kann in An- oder Abwesenheit eines Lösungsmittels erfolgen.

Die gegebenenfalls verwendeten Lösungsmittel müssen gegenüber den Reaktionspartnern inert sein und diese lösen können. So eignen sich beispielsweise aliphatische oder aromatische Kohlenwasserstoffe, wie Hexan, Benzol, Toluol oder Xylol, oder chlorierte Kohlenwasserstoffe, wie Dichlormethan oder Chlorbenzol, sowie Ether, wie Dioxan oder Diethylether, oder aprotische Lösungsmittel, wie Dimethylformamid, für diese Aufgabe. Die Reaktionstemperatur bewegt sich im allgemeinen je nach Reaktionsführung und Reaktionspartner im Temperaturbereich von —10 °C bis 250 °C.

Vorzugsweise führt man die Umsetzungen des Allylphenols mit der Thiocarbonsäure zwischen 40 und 80 °C unter Schutzgas, beispielsweise $N_2$, in Abwesenheit eines Lösungsmittels aus. Als Radikalbildner bei dieser Variante setzt man zweckmässig AIBN ein, obgleich auch andere Radikalbildner sich für diesen Zweck eignen. Anschliessend hydrolysiert man mit wässriger Kalilauge.

Die erfindungsgemässen Phenole können aus dem Reaktionsgemisch in der üblichen Weise isoliert werden. Sie lassen sich beispielsweise durch Destillation, fraktionierte Kristallisation oder Extraktion entfernen, wobei die Extraktion vorzugsweise mit einer wässrigen alkalischen Lösung durchgeführt wird.

Die Verbindungen der Formel I und II lassen sich als Härter für Epoxidharze verwenden.

Die Erfindung betrifft daher auch Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes

b) mindestens eine Verbindung der Formel I und/oder II, und

c) gegebenenfalls Härtungsbeschleuniger ; sowie die daraus durch Erwärmen erhältlichen gehärteten Produkte.

Bevorzugt werden Zusamensetzungen enthaltend a) das Epoxidharz, und b) mindestens eine Verbindung der Formel I.

Die zu verwendeten Epoxidharze haben vorzugsweise im Durchschnitt mehr als eine Epoxidgruppe pro Molekül. Insbesondere seien genannt : Alicyclische Polyepoxide, wie Epoxyethyl-3,4-epoxycyclohexan (Vinyl-cyclohexendiepoxid), Limonendiepoxid, Dicyclopentadiendiepoxid, Bis(3,4-epoxycyclohexylmethyl)adipat, 3',4'-Epoxycyclohexylmethyl-3,4-epoxycyclohexancarboxylat, 3',4'-Epoxy-6'-methylcyclohexylmethyl-3,4-epoxy-6-methylcyclohexancarboxylat, 3-(3',4'-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.

Di- oder Polyglycidylether von mehrwertigen Alkoholen, wie 1,4-Butandiol oder Polyalkylenglykolen, wie Polypropylenglykole, Di- oder Polyglycidylether von cycloaliphatischen Polyolen, wie 2,2-Bis(4'-hydroxycyclohexyl)propan, Di- oder Polyglycidylether von mehrwertigen Phenolen, wie Resorcin, Bis(4-hydroxyphenyl)methan (Bisphenol F), 2,2-Bis(4'-hydroxyphenyl)propan (Bisphenol A), 2,2-Bis(4'-hydroxy-3'-5'-dibromphenyl)propan, 1,1,2,2-Tetrakis(4'-hydroxyphenyl)ethan, oder unter sauren Bedingungen erhaltene Kondensationsprodukte von Phenolen mit Formaldehyd, wie Phenol-Novolake und Kresol-Novolake ; ferner Di- oder Poly(β-methylglycidyl)ether der oben angeführten Polyalkohole und Polyphenole.

Polyglycidylester und Poly(β-methylglycidyl)ester von mehrwertigen Carbonsäuren, wie Phthalsäure, Terephthalsäure, Tetrahydrophthalsäure und Hexahydrophthalsäure.

N-Glycidylderivate von Aminen, Amiden und heterocyclischen Stickstoffbasen, wie N,N-Diglycidylanilin, N,N-Diglycidyltoluidin, N,N,N',N'-Tetraglycidyl-bis(4-aminophenyl)methan, Triglycidylisocyanurat, N,N'-Diglycidylethylharnstoff, N,N'-Diglycidyl-5,5-dimethylhydantoin, N,N'-Diglycidyl-5-isopropylhydantoin, N,N'-Diglycidyl-5,5-dimethyl-6-isopropyl-5,6-dihydrouracil.

Besonders bevorzugt sind Polyglycidylether von Phenol- oder Kresol-Formaldehyd Novolaken sowie Diglycidylether von Bisphenol A und Bisphenol F.

Geeignete Katalysatoren (Beschleuniger) sind z. B. basische organische Verbindungen, wie primäre und/oder sekundäre Amine, beispielsweise N,N-Dimethyl-1,7-diamino-4-aza-heptan, oder tertiäre Amine, deren Salze oder quaternäre Ammoniumverbindungen, z. B. Benzyldimethylamin, 2,4,6-Tris(dimethylaminomethyl)phenol, 1-Methyl-imidazol, 2-Ethyl-4-methylimidazol, 2-Phenylimidazol, 4-Aminopyridin, Tripentylammoniumphenolat ; oder Alkalimetallalkoholate, wie z. B. Natriumhexantriolat.

Insbesondere in Kombination mit starken organischen Basen, wie beispielsweise Tetrabutylammoniumhydroxid, lassen sich die Verbindungen I und II zur Tieftemperaturhärtung von Epoxidharzen einsetzen.

Die Umsetzung (Härtung) der erfindungsgemässen Mischungen wird zweckmässig im Temperaturintervall von —25 °C bis 200 °C, bevorzugt von 25-180 °C, durchgeführt.

Bevorzugte Härtungsbeschleuniger sind 2-Phenylimidazol, N,N-Dimethylbenzylamin oder 2,4,6-Tris(dimethylaminomethyl)phenol.

Man kann die Härtung in bekannter Weise auch zwei- oder mehrstufig durchführen, wobei die erste Härtungsstufe bei niedriger Temperatur (Raumtemperatur) und die Nachhärtung bei höherer Temperatur durchgeführt wird.

In der Regel wird eine zweistufige Härtung in der Weise durchgeführt, dass die Härtungsreaktion

zunächst vorzeitig abgebrochen bzw. die erste Stufe bei Raumtemperatur oder wenig erhöhter Temperatur durchgeführt wird, wobei ein noch schmelzbares und/oder lösliches, härtbares Vorkondensat (sogenannte « B-Stufe ») aus der Epoxid-Komponente (a) und der Härterkomponente (b) erhalten wird. Ein derartiges Vorkondensat kann z. B. zur Herstellung von « Prepregs », Pressmassen oder Sinterpulvern dienen.

Die erfindungsgemässen Zusammensetzungen enthaltend a) ein Epoxidharz mit durchschnittlich mehr als einer Epoxidgruppe im Molekül, und b) mindestens eine Verbindung der Formel I oder II bilden schmelzbare B-Stufen, wenn diese Harz/Härter Mischungen bei Raumtemperatur gelagert werden. Solche Mischungen sind für eine gewisse Zeit (Tage) lagerfähig und können dann in geeigneter Weise weiterverarbeitet werden.

Der Ausdruck « Härten », wie er hier gebraucht wird bedeutet die Umwandlung der löslichen, entweder flüssigen oder schmelzbaren Epoxidharze in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die erfindungsgemässen Gemische können durch einfaches Zusammenrühren der Komponenten und vorsichtiges Aufwärmen bis zum Lösen der Komponenten hergestellt werden. Falls ein festes Epoxidharz vorliegt, wird dieses vorübergehend zum Schmelzen erwärmt, worauf man darin den Härter und gegebenenfalls den Härtungsbeschleuniger und/oder weitere Zusätze löst.

Die erfindungsgemässen härtbaren Mischungen können ferner vor der Härtung in irgendeiner Phase mit üblichen Modifizierungsmitteln, wie Streck-, Füll- und Verstärkungsmitteln, Pigmenten, Farbstoffen, Weichmachern, Verlaufmitteln, Thioxotropiermitteln, Flexibilisatoren, flammhemmenden Stoffen oder Formtrennmitteln versetzt werden.

Als Streckmittel, Verstärkungsmittel, Füllmittel und Pigmente, die in den erfindungsgemässen härtbaren Mischungen eingesetzt werden können, seien z. B. genannt : Steinkohlenenteer, Bitumen, flüssige Cumaron-Inden-Harze, Textilfasern, Glasfasern, Asbestfasern, Borfasern, Kohlenstoffasern, Cellulose, Polyester, Polyamide, Polyethylenpulver, Polypropylenpulver, Holzmehl, Quarzmehl, mineralische Silikate, wie Glimmer, Asbestmehl, Schiefermehl, Kaolin, Kieselsäureaerogel, Lithopone, Schwerspat, Titandioxid, Russ, Graphit, Oxidfarben, wie Eisenoxid, oder Metallpulver, wie Aluminiumpulver oder Eisenpulver.

Als Weichmacher können für die Modifizierung der härtbaren Mischungen z. B. Dibutyl-, Dioctyl- oder Dinonylphthalat, Trikresylphosphat, Trixylenylphosphat und Diphenoxyethylformal eingesetzt werden.

Als Verlaufmittel beim Einsatz der härtbaren Mischungen speziell im Oberflächenschutz, kann man z. B. Silikone, flüssige Acrylharze, Celluloseacetobutyrat, Polyvinylbutyral, Wachse, Stearate etc. (welche z. T. auch als Formtrennmittel Anwendung finden) zusetzen.

Als Flexibilisatoren eignen sich z. B. Oligoestersegmente, Polyester, Thermoplaste und Butadien-Acrylnitril-Oligomere, wie Hycar® von der Firma Goodrich.

Die erfindungsgemäss gehärteten Gemische zeichnen sich insbesondere durch gute Haftung auf Metallen, hohe Wärmeformbeständigkeit, sowie gute Feuchtigkeits- und Chemikalien-Beständigkeit aus.

Die erfindungsgemässen härtbaren Gemische finden ihren Einsatz vor allem auf den Gebieten des Oberflächenschutzes, der Elektrotechnik, der Laminierverfahren, der Klebstofftechnik und im Bauwesen. Sie können in jeweils dem speziellen Anwendungszweck angepasster Formulierung, im ungefüllten oder gefüllten Zustand, gegebenenfalls in Form von Lösungen der Emulsionen, als Anstrichmittel, lösungsmittelfreie Beschichtungen, Sinterpulver, Pressmassen, Spritzgussformulierungen, Tauchharze, Giessharze, Schaumstoffe, Klebstoffe, Filme, Folien, Bindemittel, Werkzeugharze, Laminierharze, Dichtungs- und Spachtelmassen, Bodenbelegsmassen und Bindemittel für mineralische Aggregate verwendet werden.

Insbesondere betrifft die Erfindung die Verwendung von Mischungen enthaltend a) ein Epoxidharz mit durchschnittlich mehr als einer Epoxidgruppe im Molekül, und b) mindestens eine Verbindung der Formel I und/oder II als Klebstoffe.

## Herstellungsbeispiele

1. Herstellung von 2,2'-Bis-(3-(3-mercaptopropyl)-4-hydroxyphenyl)-propan

1a) o,o'-Bis-(3-acetylthiopropyl)-bisphenol A

666,8 g 2,2'-Bis-(3-allyl-4-hydroxyphenyl)-propan werden in einem Reaktionskolben, ausgestattet mit Rührer, Tropftrichter und Stickstoffeinlassrohr vorgelegt und unter Stickstoffstrom auf 75 °C erwärmt. Es werden 4,92 g AIBN zugefügt und anschliessend 780 g Thioessigsäure im Zeitraum einer Stunde zugetropft. Die Temperatur im Reaktionskolben wird bei 75 °C gehalten und alle 20 Minuten werden weitere 4,92 g AIBN zugegeben, bis die Gesamtmenge 19,68 g erreicht. Nach dem Zutropfen der Thioessigsäure wird weitere 4 Stunden bei 75 °C gerührt. Dann wird nochmals eine Portion von 4,92 g AIBN zugegeben und für weitere 3 Stunden gerührt.

Anschliessend wird die Reaktionsmischung im Rotationsverdampfer eingeengt. Man erhält 1 036 g einer gelben Paste.

a) Elementaranalyse :
berechnet : C 65,19 H 7,00 S 13,92 %
gefunden : C 64,5 H 6,96 S 13,2 %

b) 100 MHz $^1$H-NMR Spektrum.

Man findet keine olefinischen Protonensignale in der Region zwischen 5 und 6 ppm (Standard : TMS). Es sind also keine Allylgruppen mehr nachweisbar.

Signale bei :
2,3 ppm (2 Protonen ; $—S—CO—CH_3$)
1,8 ppm (2 Protonen ; $—S—C—CH_2—C—$Phenyl)
2,6 ppm (2 Protonen ; $—S—CH_2—C—C—$Phenyl)
2,8 ppm (2 Protonen ; $—S—C—C—CH_2—$Phenyl).

1b) Reinigung des Rohprodukts von Beispiel 1a

360,8 g des pastenförmigen Produktes von Beispiel 1a) werden in 100 ml heissem Xylol gelöst. Es wird 1 g Aktivkohle zugegeben, die Lösung filtriert und anschliessend bei 5 °C stehen gelassen. Die ausgefallenen Kristalle werden abfiltriert und bei 100 °C im Vakuum getrocknet (50 mbar). Man erhält 142 g weisse Kristalle vom F.P. 118,1-119,5 °C.

Elementaranalyse :
berechnet : C 65,19 H 7,00 S 13,92 O 13,89 %
gefunden : C 65,88 H 7,07 S 13,43 O 13,79 %

1c) o,o'-Bis-(3-mercaptopropyl)-bisphenol A

50 g des in 1a) hergestellten und in 1b) gereinigten Produktes werden zu einer Lösung von 44,1 g KOH in 200 ml einer Ethanol-Wassermischung (50 : 50 Vol. Teile) gegeben. Die Mischung wird 1 Stunde lang bei 50 °C gehalten und dann eine Stunde bei ca. 80 °C unter Rückfluss gekocht. Nach Abkühlen auf Raumtemperatur wird die Mischung mit 37,5 %iger Salzsäure auf einen pH Wert von 6-7 eingestellt und mit 1 Liter Dichlormethan ausgeschüttelt. Die organische Phase wird abgetrennt, über Natriumsulfat getrocknet und das Lösungsmittel im Rotationsverdampfer abgezogen. Man erhält 36,4 g einer leicht gelben harzartigen Flüssigkeit (Ausbeute : 38,9 g d. Th., bezogen auf acyliertes Produkt).

Analysedaten

a) Elementaranalyse
berechnet : C 66,98 H 7,49 %
gefunden : C 67,2 H 7,42 %

b) Thiolgehalt (bestimmt durch iodometrische Titration)
4,60 -SH Aequivalente/kg
c) $^1$H-NMR Spektrum (250 MHz)

| | |
|---|---|
| 6,5-7 ppm (m) | 6 aromatische Protonen |
| 5,2 ppm (s) | 2 phenolische Protonen |
| 2,5-2,9 ppm (m) | 8 aliphatische Protonen ($—CH_2—CH_2$-Phenyl) |
| 1,75-2,1 ppm (m) | 4 aliphatische Protonen ($—C—CH_2—S—$) |
| 1,6 ppm (s) | 6 aliphatische Protonen ($—C(CH_3)_2—$) |
| 1,4 ppm (m) | 2 Thiol-Protonen |

2. Herstellung eines Novolaks auf Basis von Formaldehyd und 2-Mercaptopropylphenol

2a) Herstellung eines (o-Acetylthiopropyl)-phenol Formaldehyd Novolaks

Nach dem Verfahren gemäss Beispiel 1a) werden 419,9 g eines 2-Allylphenol Formaldehyd Novolaks (hergestellt durch Kondensation von 1 Teil Formaldehyd mit 6 Teilen 2-Allylphenol ; Allylgehalt : 2,36 Val) mit 179,9 g Thioessigsäure (2,36 Mol) und 10,2 g AIBN unter Stickstoff bei 80 °C umgesetzt. Das AIBN wird gemäss Beispiel 1 in fünf Portionen zu jeweils 2,04 g zugegeben. Man erhält 589,2 g eines Produktes (Ausbeute : 98,2 % der Theorie).

Die $^1$H NMR Signale (250 MHz) der Allylprotonen des Ausgangsproduktes im Bereich von 5,1-5,2 ppm bzw. 5,9-6,1 ppm (gegen TMS) sind im $^1$H NMR Spektrum des Endproduktes verschwunden. Dafür tritt ein Signal der Protonen der Acetylgruppe auf (bei 2,3 ppm).

2b) Reinigung des Rohproduktes

177,2 g des Rohproduktes von Beispiel 2a) werden mit 200 ml Dichlormethan und 220 ml wässrigem Ethanol (Wassergehalt: 30 Vol.%) versetzt und die überstehende Phase abdekantiert. Es werden nochmals 220 ml wässriges Ethanol und 100 ml Wasser zugegeben und abdekantiert.

Der zurückbleibende Novolak wird noch zweimal mit 230 ml Wasser gewaschen und über $Na_2SO_4$ getrocknet. Anschliessend trocknet man das Produkt noch bei 40 °C (17 mm Hg) bzw. 60 °C (3 mm Hg) am Rotationsdampfer. Man erhält 164 g eines gereinigten Endproduktes. Dieses Produkt besitzt ein GPC-analytisch bestimmtes Molekulargewicht von 527 (Zahlenmittel) bzw. 597 (Gewichtsmittel).

2c) Verseifung des acetylierten Produktes zum Mercaptan

50 g des gemäss 2a) hergestellten und gemäss 2b) gereinigten Produktes werden in einem Reaktionskolben vorgelegt. Dazu gibt man eine Lösung von 22,4 g (0,4 Mol) Kaliumhydroxid in 200 ml Ethanol-/Wassermischung (50 : 50 Vol. Teile). Die Mischung wird etwa eineinhalb Stunden bei ca. 50 °C reagieren gelassen und anschliessend noch zwei Stunden unter Rückfluss gekocht. Nach dem Abkühlen auf Raumtemperatur wird die Mischung mit 15 ml 37,5 %iger Salzsäure neutralisiert, der Niederschlag abfiltriert, mit 350 ml Dichlormethan extrahiert und über $Na_2SO_4$ getrocknet. Nach dem Abfiltrieren wird das Lösungsmittel abgezogen. Man erhält 37,65 g eines viskosen harzartigen Produktes.

Mercaptangehalt (titrimetrisch bestimmt): 4,04 Aequ./kg
(Theorie: 4,07 Aequ./Kg).
Molekulargewicht (GPC-analytisch bestimmt): $\overline{M}_n = 484$;
$\overline{M}_w = 569$.

## Anwendungsbeispiele

### Beispiel A

Es wird eine Klebstofformulierung bestehend aus 100 Gew.-Teilen eines flüssigen Epoxidharzes auf Basis von Bisphenol A (Epoxidwert: 5,25 Val/kg), 49,8 Gew.-Teilen des Härters von Beispiel 1c) und 0,02 Gew.-Teilen 2-Phenylimidazol als Härtungsbeschleuniger durch Vermischen der Komponenten bei 60 °C hergestellt.

Mit dieser Formulierung werden Klebverbindungen zwischen Aluminiumflächen hergestellt. Dazu werden Ausbohrungen in einer Aluminiumplatte, dei einen definierten Durchmesser und eine definierte Tiefe besitzen, mit der Harzmischung gefüllt. Anschliessend werden Aluminiumzylinder definierten Durchmessers auf dieser Unterlage fixiert.

Die Klebstellen werden jeweils 2 Stunden bei 120 °C, 150 °C und 180 °C gehärtet.

Die Messung der Adhäsion der Klebstelle erfolgt mittels eines Twistometers (vgl. Adhesion 3, edited by K.W. Allen; Applied Science Publishers Ltd.; Barking (Essex); 1978).

Dazu wird die Al-Grundplatte fixiert und auf den Al-Zylinder mittels eines Hebelarmes eine definierte Torsionskraft ausgeübt. Aus der maximalen Torsionskraft, die zum Bruch der Klebstelle führt, lässt sich die Adhäsion ermitteln.

Man misst eine Adhäsion von 70,1 N/mm².

### Beispiel B

Eine Klebstofformulierung bestehend aus 100 Gew.-Teilen Flüssigepoxidharz (Diglycidylether auf Bisphenol A Basis; Epoxidgehalt; 5,3 Val/kg), 41,8 Gew.-Teilen 2,2'-Bis-(3-mercaptopropyl)-bisphenol A gemäss Beispiel 1c) und 2,2 Gew.-Teilen N,N-Dimethyl-1,7-diamino-4-azaheptan als Härtungsbeschleuniger wird durch Vermischen der Komponenten bei Raumtemperatur hergestellt. Mit dieser Formulierung werden Al/Al Verklebungen hergestellt. Die Härtung der Klebverbindungen erfolgt durch 30-minütiges Erhitzen auf 180 °C.

Als Qualitätskriterium der Klebverbindung wird die Zugscherfestigkeit nach DIN 53283 (auf Anticorodal B) ermittelt. Man misst einen Wert von 22,4 N/mm².

### Beispiel C

Eine Klebstofformulierung bestehend aus 100 Gew.-Teilen Flüssigepoxidharz (Diglycidylether auf Bisphenol A Basis; Epoxidgehalt: 5,3 Val/kg), 44,0 Gew.-Teilen 2,2'-Bis-(3-mercaptopropyl)-bisphenol A gemäss Beispiel 1c) und 0,25 Gew.-Teilen 2-Phenylimidazol als Härtungsbeschleuniger wird durch Vermischen der Komponenten bei 60 °C hergestellt.

Mit dieser Formulierung werden Al/Al Verklebungen hergestellt. Die Härtung der Klebverbindungen und die Messung der Zugscherfestigkeit erfolgt wie in Beispiel B beschrieben. Man misst eine Zugscherfestigkeit von 8,6 N/mm².

**Patentansprüche** (für die Vertragsstaaten : CH, DE, FR, GB, IT, LI, NL)

1. Verbindungen der Formeln I oder II

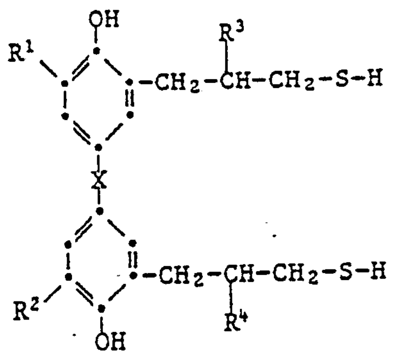

(I)

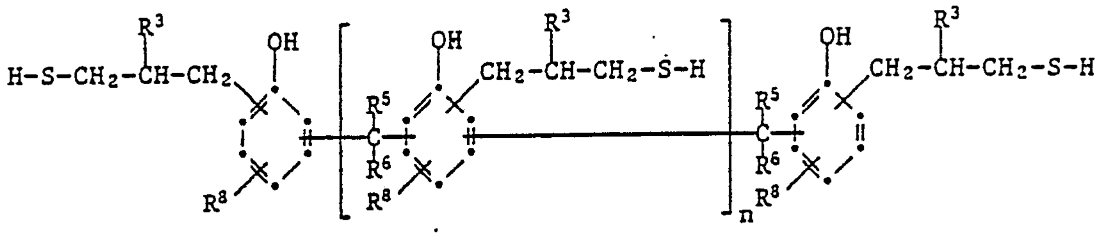

(II)

worin $R^1$ und $R^2$ unabhängig voneinander Wasserstoff, $C_1$-$C_{18}$Alkyl, $C_3$-$C_{18}$Alkenylmethyl, Cyclohexyl, Phenyl, Benzyl oder Tolyl bedeuten oder ein Rest $—CH_2—CHR^3—CH_2—S—H$ sind, $R^3$ und $R^4$ unabhängig voneinander Wasserstoff oder Methyl sind, X $—CR^5R^6—$, —S—, —SO—, $—SO_2—$ oder $—(CH_3)C[—(CH_2)_m—COOR^7]—$ bedeutet, $R^5$ und $R^6$ unabhängig voneinander Wasserstoff oder $C_1$-$C_6$Alkyl sind, $R^7$ $C_1$-$C_{18}$Alkyl ist, $R^8$ Wasserstoff, $C_1$-$C_{18}$Alkyl, $C_3$-$C_{18}$Alkenylmethyl oder Phenyl darstellt, m 1 oder 2 bedeutet und n eine ganze Zahl von 1 bis 10 ist.

2. Verbindungen der Formel I oder II gemäss Anspruch 1, worin $R^3$ und $R^4$ Wasserstoff bedeuten.

3. Verbindungen der Formel I gemäss Anspruch 1, worin $R^1$ und $R^2$ unabhängig voneinander Wasserstoff oder $C_1$-$C_{12}$Alkyl sind, $R^3$ und $R^4$ Wasserstoff bedeuten, X eine Gruppe $—CR^5R^6—$ darstellt und worin $R^5$ und $R^6$ unabhängig voneinander Wasserstoff oder Methyl sind.

4. Verbindungen der Formel I gemäss Anspruch 1, worin $R^1$, $R^2$, $R^3$ und $R^4$ Wasserstoff sind, und X eine Gruppe $—CH_2—$, $—CH(CH_3)—$ oder $—C(CH_3)_2—$ darstellt.

5. Verbindungen der Formel I gemäss Anspruch 1, worin $R^1$ und $R^2$ eine Gruppe $—CH_2—CH(R^3)—CH_2—S—H$ darstellen.

6. Verbindungen der Formel II gemäss Anspruch 1, worin $R^3$ Wasserstoff ist, $R^8$ Wasserstoff oder Methyl bedeutet, $—CR^5R^6—$ eine Gruppe $—CH_2—$, $—CH(CH_3)—$ oder $—C(CH_3)_2—$ darstellt und worin n eine ganze Zahl von 1 bis 4 ist.

7. Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes

b) mindestens eine Verbindung der Formel I und/oder II gemäss Anspruch 1, und

c) gegebenenfals einen Härtungsbeschleuniger.

8. Gehärtete Produkte erhältlich durch Erwärmen von Zusammensetzungen gemäss Anspruch 7.

9. Verwendung der Zusammensetzungen gemäss Anspruch 7 als Klebstoffe.

**Patentansprüche** (für den Vertragsstaat AT)

1. Verfahren zur Herstellung von Verbindungen der Formeln I oder II

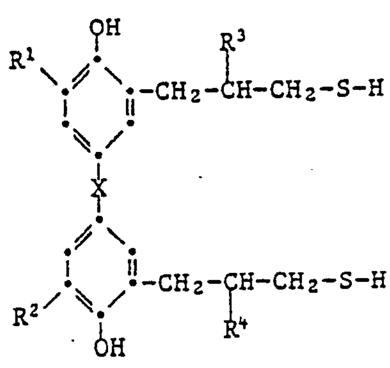

(I)

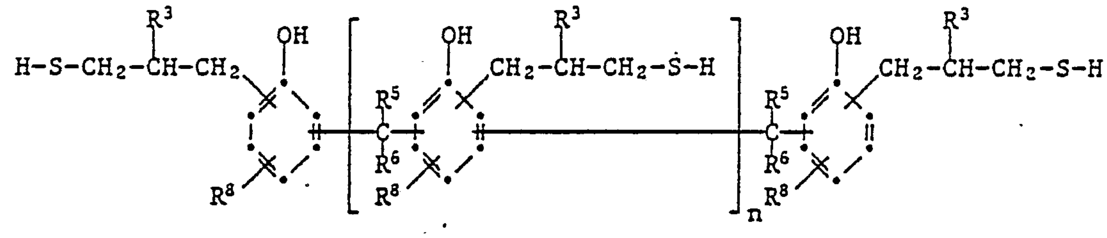

(II)

worin $R^1$ und $R^2$ unabhängig voneinander Wasserstoff, $C_1$-$C_{18}$Alkyl, $C_3$-$C_{18}$Alkenylmethyl, Cyclohexyl, Phenyl, Benzyl oder Tolyl bedeuten oder ein Rest $-CH_2-CHR^3-CH_2-S-H$ sind, $R^3$ und $R^4$ unabhängig voneinander Wasserstoff oder Methyl sind, X $-CR^5R^6-$, $-S-$, $-SO-$, $-SO_2-$ oder $-(CH_3)C[-(CH_2)_m-COOR^7]-$ bedeutet, $R^5$ und $R^6$ unabhängig voneinander Wasserstoff oder $C_1$-$C_6$Alkyl sind, $R^7$ $C_1$-$C_{18}$Alkyl ist, $R^8$ Wasserstoff, $C_1$-$C_{18}$Alkyl, $C_3$-$C_{18}$Alkenylmethyl oder Phenyl darstellt, m 1 oder 2 bedeutet und n eine ganze Zahl von 1 bis 10 ist, durch Umsetzung der Verbindungen der Formeln III oder IV

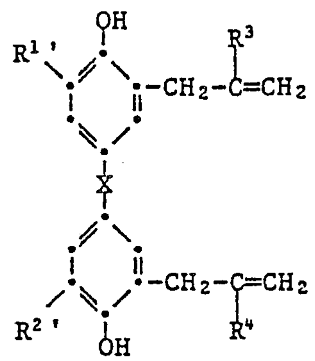

(III)

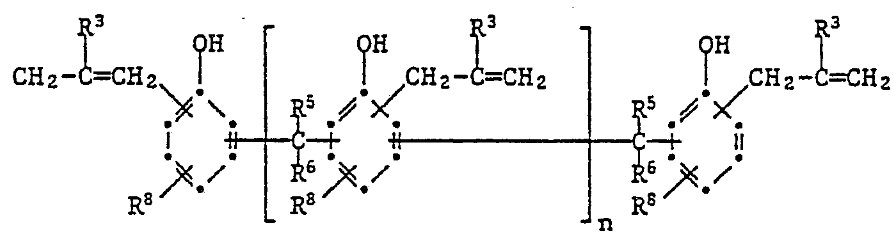

(IV)

worin $R^3$, $R^4$, $R^5$, $R^6$, $R^8$, X und n die oben definierte Bedeutung besitzen und $R^{1'}$ und $R^{2'}$ die Bedeutungen von $R^1$ und $R^2$ ausgenommen $-CH_2-CHR^3-CH_2-SH$ besitzen aber zusätzlich $-CH_2-CR^3=CH_2$ sein können mit

a) einem dem Gehalt an allylständigen Doppelbindungen im wesentlichen entsprechenden molaren Anteil an Thiocarbonsäuren der Formel V

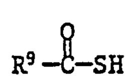

(V)

worin $R^9$ ein einwertiger organischer Rest ist und anschliessende Verseifung der Umsetzungsprodukte oder

b) durch Umsetzung der Verbindungen der Formel III oder IV mit Schwefelwasserstoff.

2. Verfahren gemäss Anspruch 1, worin $R^3$ und $R^4$ Wasserstoff bedeuten.

3. Verfahren zur Herstellung von Verbindungen der Formel I gemäss Anspruch 1, worin $R^1$ und $R^2$ unabhängig voneinander Wasserstoff oder $C_1$-$C_{12}$Alkyl sind, $R^3$ und $R^4$ Wasserstoff bedeuten, X eine Gruppe $-CR^5R^6-$ darstellt und worin $R^5$ und $R^6$ unabhängig voneinander Wasserstoff oder Methyl sind.

4. Verfahren zur Herstellung von Verbindungen der Formel I gemäss Anspruch 1, worin $R^1$, $R^2$, $R^3$ und $R^4$ Wasserstoff sind, und X eine Gruppe $-CH_2-$, $-CH(CH_3)-$ oder $-C(CH_3)_2-$ darstellt.

5. Verfahren zur Herstellung von Verbindungen der Formel I gemäss Anspruch 1, worin $R^1$ und $R^2$ eine Gruppe $-CH_2-CH(R^3)-CH_2-S-H$ darstellen.

6. Verfahren zur Herstellung von Verbindungen der Formel II gemäss Anspruch 1, worin $R^3$

Wasserstoff ist, $R^8$ Wasserstoff oder Methyl bedeutet, $—CR^5R^6—$ eine Gruppe $—CH_2—$, $—CH(CH_3)—$ oder $—C(CH_3)_2—$ darstellt und worin n eine ganze Zahl von 1 bis 4 ist.

7. Zusammensetzungen enthaltend

a) ein Epoxidharz mit durchschnittlich mehr als einer Epoxidgruppe im Molekül oder ein noch schmelzbares und/oder lösliches härtbares Vorkondensat des Epoxidharzes

b) mindestens eine Verbindung der Formel I und/oder II gemäss Anspruch 1, und

c) gegebenenfalls einen Härtungsbeschleuniger.

8. Gehärtete Produkte erhältlich durch Erwärmen von Zusammensetzungen gemäss Anspruch 7.

9. Verwendung der Zusammensetzungen gemäss Anspruch 7 als Klebstoffe.

**Claims** (for the Contracting States : CH, DE, FR, GB, IT, LI, NL)

1. A compound of formula I or II

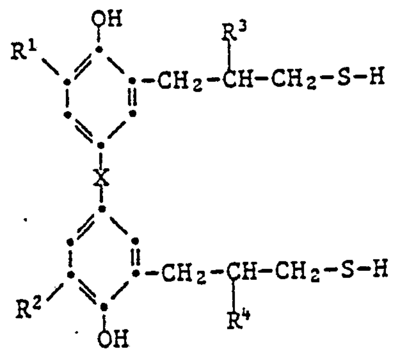

(I)

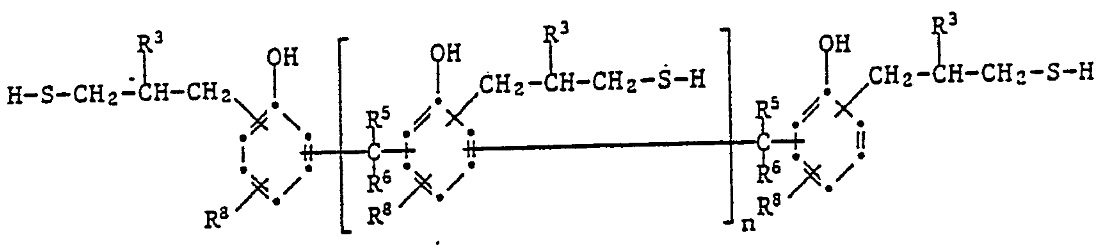

(II)

wherein $R^1$ and $R^2$ are each independently of the other hydrogen, $C_1$-$C_{18}$alkyl, $C_3$-$C_{18}$alkenylmethyl, cyclohexyl, phenyl, benzyl or tolyl, or are a $—CH_2—CHR^3—CH_2—S—H$ radical, $R^3$ and $R^4$ are each independently of the other hydrogen or methyl, X is $—CR^5R^6—$, $—S—$, $—SO—$, $—SO_2—$ or $—(CH_3)C[—(CH_2)_m—COOR^7]—$, $R^5$ and $R^6$ are each independently of the other hydrogen or $C_1$-$C_6$alkyl, $R^7$ is $C_1$-$C_{18}$alkyl, $R^8$ is hydrogen, $C_1$-$C_{18}$alkyl, $C_3$-$C_{18}$alkenylmethyl or phenyl, m is 1 or 2 and n is an integer from 1 to 10.

2. A compound of formula I or II according to claim 1, wherein $R^3$ and $R^4$ are hydrogen.

3. A compound of formula I according to claim 1, wherein $R^1$ and $R^2$ are each independently of the other hydrogen or $C_1$-$C_{12}$alkyl, $R^3$ and $R^4$ are hydrogen, X is a $—CR^5R^6—$ group, and wherein $R^5$ and $R^6$ are each independently of the other hydrogen or methyl.

4. A compound of formula I according to claim 1, wherein $R^1$, $R^2$, $R^3$ and $R^4$ are hydrogen, and X is a $—CH_2—$, $—CH(CH_3)—$ or $—C(CH_3)_2—$ group.

5. A compound of formula I according to claim 1, wherein $R^1$ and $R^2$ are a $—CH_2—CH(R^3)—CH_2—S—H$ group.

6. A compound of formula II according to claim 1, wherein $R^3$ is hydrogen, $R^8$ is hydrogen or methyl, $—CR^5R^6—$ is a $—CH_2—$, $—CH(CH_3)—$ or $—C(CH_3)_2—$ group, and wherein n is an integer from 1 to 4.

7. A composition containing

a) an epoxy resin having on average more than one epoxy group in the molecule or a still fusible and/or soluble curable precondensate of the epoxy resin, and

b) at least one compound of formula I and/or II according to claim 1, and

c) optionally, a curing accelerator.

8. A cured product obtainable by heating a composition according to claim 7.

9. The use of a composition according to claim 7 as an adhesive.

**Claims** (for the Contracting State AT)

1. A process for the preparation of a compound of formula I or II

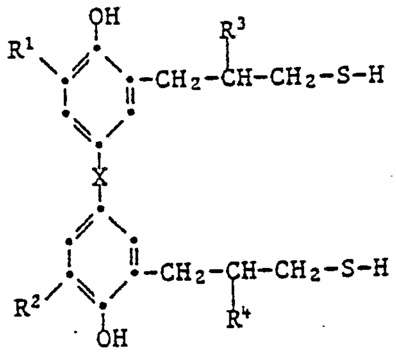

(I)

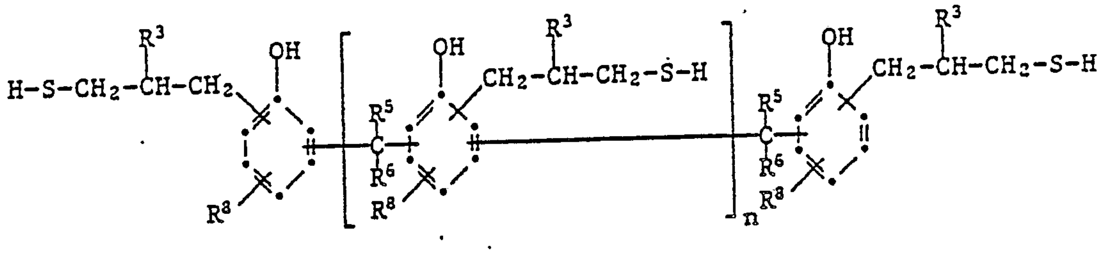

(II)

wherein $R^1$ and $R^2$ are each independently of the other hydrogen, $C_1$-$C_{18}$alkyl, $C_3$-$C_{18}$alkenylmethyl, cyclohexyl, phenyl, benzyl or tolyl, or are a $-CH_2-CHR^3-CH_2-S-H$ radical, $R^3$ and $R^4$ are each independently of the other hydrogen or methyl, X is $-CR^5R^6-$, $-S-$, $-SO-$, $-SO_2-$ or $-(CH_3)C[-(CH_2)_m-COOR^7]-$, $R^5$ and $R^6$ are each independently of the other hydrogen or $C_1$-$C_6$alkyl, $R^7$ is $C_1$-$C_{18}$alkyl, $R^8$ is hydrogen, $C_1$-$C_{18}$alkyl, $C_3$-$C_{18}$alkenylmethyl or phenyl, m is 1 or 2 and n is an integer from 1 to 10, by reaction of the compounds of formula III or IV

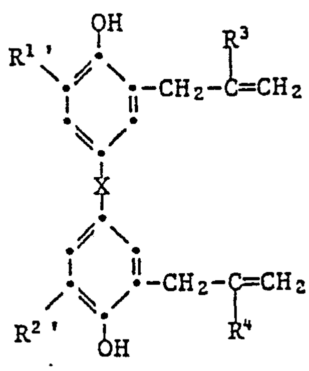

(III)

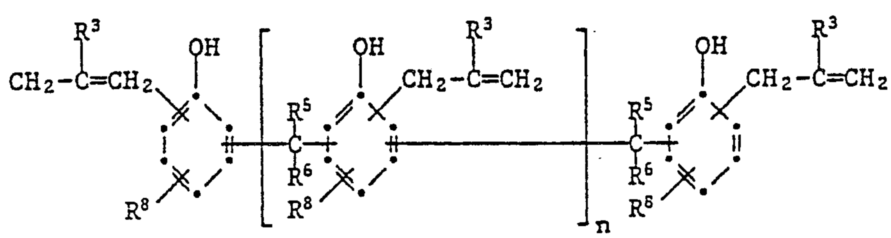

(IV)

wherein $R^3$, $R^4$, $R^5$, $R^6$, $R^8$, X and n have the meanings as defined above and $R^{1'}$ and $R^{2'}$ have the meanings of $R^1$ and $R^2$ except for $-CH_2-CHR^3-CH_2-SH$ but they can additionally be $-CH_2-CR^3=CH_2$, with

a) a molar amount of thiocarboxylic acid of formula V

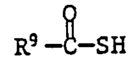 (V)

wherein $R^9$ is a monovalent organic radical, which amount is substantially proportional to the content of allylic double bonds, followed by hydrolysis of the reaction products, or

b) by reaction of the compounds of formula III or IV with hydrogen sulphide.

2. A process according to claim 1, wherein $R^3$ and $R^4$ are hydrogen.

3. A process for the preparation of a compound of formula I according to claim 1, wherein $R^1$ and $R^2$ are each independently of the other hydrogen or $C_1$-$C_{12}$alkyl, $R^3$ and $R^4$ are hydrogen, X is a $—CR^5R^6—$ group, and wherein $R^5$ and $R^6$ are each independently of the other hydrogen or methyl.

4. A process for the preparation of a compound of formula I according to claim 1, wherein $R^1$, $R^2$, $R^3$ and $R^4$ are hydrogen, and X is a $—CH_2—$, $—CH(CH_3)—$ or $—C(CH_3)_2—$ group.

5. A process for the preparation of a compound of formula I according to claim 1, wherein $R^1$ and $R^2$ are a $—CH_2—CH(R^3)—CH_2—S—H$ group.

6. A process for the preparation of a compound of formula II according to claim 1, wherein $R^3$ is hydrogen, $R^8$ is hydrogen or methyl, $—CR^5R^6$ is a $—CH_2—$, $—CH(CH_3)—$ or $—C(CH_3)_2—$ group, and wherein n is an integer from 1 to 4.

7. A composition containing

a) an epoxy resin having on average more than one epoxy group in the molecule or a still fusible and/or soluble curable precondensate of the epoxy resin, and

b) at least one compound of formula I and/or II according to claim 1, and

c) optionally, a curing accelerator.

8. A cured product obtainable by heating a composition according to claim 7.

9. The use of a composition according to claim 7 as an adhesive.

**Revendications** (pour les Etats contractants : CH, DE, FR, GB, IT, LI, NL)

1. Composés répondant à l'une des formules I et II :

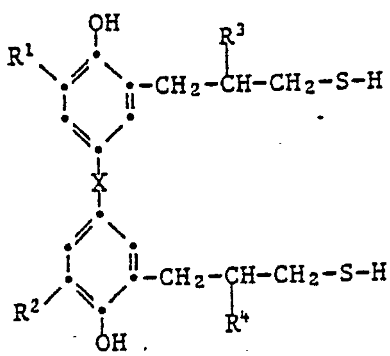 (I)

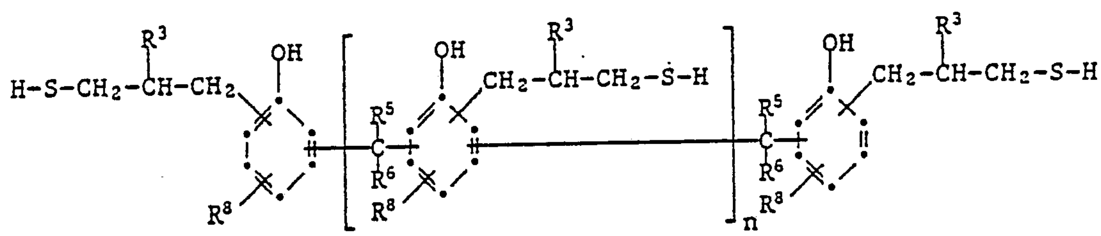 (II)

dans lesquelles $R^1$ et $R^2$ représentent chacun, indépendamment l'un de l'autre, l'hydrogène, un alkyle en $C_1$-$C_{18}$, un alcénylméthyle en $C_3$-$C_{18}$, un cyclohexyle, un phényle, un benzyle ou un tolyle, ou encore un radical $—CH_2—CHR^3—CH_2—S—H$, $R^3$ et $R^4$ représentent chacun, indépendamment l'un de l'autre, l'hydrogène ou un méthyle, X représente un radical $—CR^5R^6—$, $—S—$, $—SO—$, $—SO_2—$ ou $—(CH_3)C[—(CH_2)_m—COOR^7]—$, $R^5$ et $R^6$ représentent chacun, indépendamment l'un de l'autre, l'hydrogène ou un alkyle en $C_1$-$C_6$, $R^7$ représente un alkyle en $C_1$—$C_{18}$, $R^8$ représente l'hydrogène, un alkyle en $C_1$-$C_{18}$, un alcénylméthyle en $C_3$-$C_{18}$ ou un phényle, m représente le nombre 1 ou le nombre 2, et n représente un nombre entier de 1 à 10.

2. Composés de formule I ou II selon la revendication 1, dans lesquels $R^3$ et $R^4$ représentent chacun l'hydrogène.

3. Composés de formule I selon la revendication 1 dans lesquels $R^1$ et $R^2$ représentent chacun, indépendamment l'un de l'autre, l'hydrogène ou un alkyle en $C_1$-$C_{12}$, $R^3$ et $R^4$ représentent chacun

l'hydrogène, X représente un radical —$CR^5R^6$—, et $R^5$ et $R^6$ représentent chacun, indépendamment l'un de l'autre, l'hydrogène ou un méthyle.

4. Composés de formule I selon la revendication 1 dans lesquels $R^1$, $R^2$, $R^3$ et $R^4$ représentent chacun l'hydrogène, et X représente un radical —$CH_2$—, —$CH(CH_3)$— ou —$C(CH_3)_2$—.

5. Composés de formule I selon la revendication 1 dans lesquels $R^1$ et $R^2$ représentent chacun un radical —$CH_2$—$CH(R^3)$—$CH_2$—S—H.

6. Composés de formule II selon la revendication 1 dans lesquels $R^3$ représente l'hydrogène, $R^8$ l'hydrogène ou un méthyle, —$CR^5R^6$— un radical —$CH_2$—, —$CH(CH_3)$— ou —$C(CH_3)_2$—, et n un nombre entier de 1 à 4.

7. Compositions qui contiennent :

a) une résine époxydique dont la molécule renferme en moyenne plus d'un radical époxy, ou un pré-condensat durcissable, encore fusible et/ou soluble, de la résine époxydique,

b) au moins un composé de formule I et/ou II selon la revendication 1, et.

c) éventuellement un accélérateur de durcissement.

8. Produits durcis qui peuvent être obtenus par chauffage de compositions selon la revendication 7.

9. Application des compositions selon la revendication 7 comme colles.

**Revendications** (pour l'Etat contractant AT)

1. Procédé pour préparer des composés répondant à l'une des formules I et II :

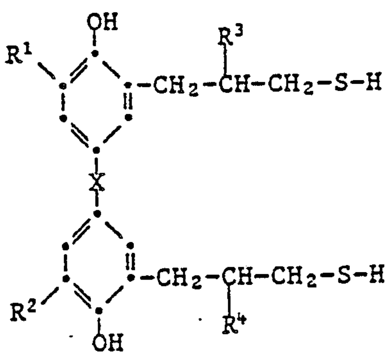

(I)

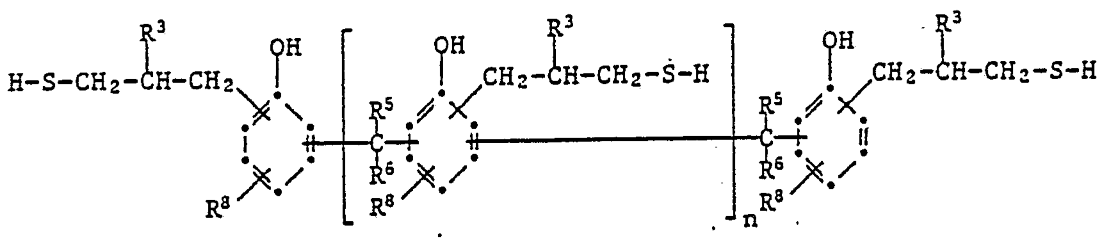

(II)

dans lesquelles $R^1$ et $R^2$ représentent chacun, indépendamment l'un de l'autre, l'hydrogène, un alkyle en $C_1$-$C_{18}$, un alcénylméthyle en $C_3$-$C_{18}$, un cyclohexyle, un phényle, un benzyle ou un tolyle, ou encore un radical —$CH_2$—$CHR^3$—$CH_2$—S—H, $R^3$ et $R^4$ représentent chacun, indépendamment l'un de l'autre, l'hydrogène ou un méthyle, X représente un radical —$CR^5R^6$—, —S—, —SO—, —$SO_2$— ou —$(CH_3)C[$—$(CH_2)_m$—$COOR^7]$—, $R^5$ et $R^6$ représentent chacun, indépendamment l'un de l'autre, l'hydrogène ou un alkyle en $C_1$-$C_6$, $R^7$ représente un alkyle en $C_1$-$C_{18}$, $R^8$ représente l'hydrogène, un alkyle en $C_1$-$C_{18}$, un alcénylméthyle en $C_3$-$C_{18}$ ou un phényle, m représente le nombre 1 ou le nombre 2, et n représente un nombre entier de 1 à 10, procédé selon lequel on fait réagir des composés répondant à l'une des formules III et IV :

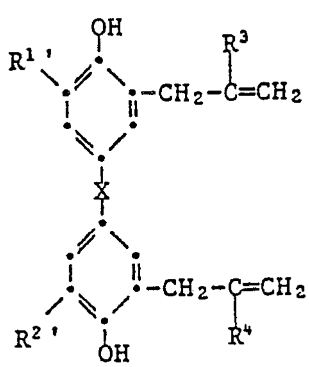

(III)

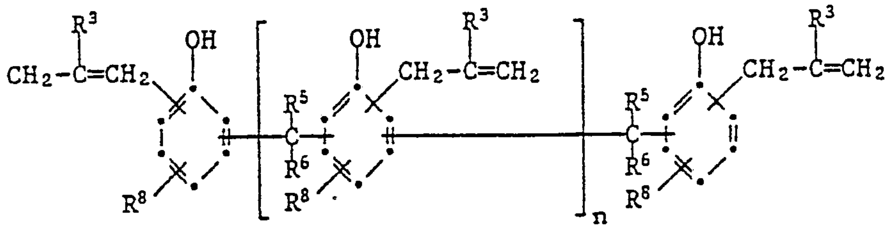 (IV)

dans lesquelles $R^3$, $R^4$, $R^5$, $R^6$, $R^8$, X et n ont les significations indiquées ci-dessus tandis que $R^{1'}$ et $R^{2'}$ ont les significations de $R^1$ et de $R^2$ à l'exception de $-CH_2-CHR^3-CH_2-SH$ mais peuvent en plus représenter un radical $-CH_2-CR^3=CH_2$, avec

a) une quantité molaire d'acides carbothioïques de formule V :

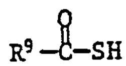 (V)

(dans cette formule, $R^9$ représente un radical organique univalent) qui correspond essentiellement à la teneur en doubles liaisons allyliques, puis on saponifie les produits réactionnels, ou

b) on fait réagir les composés de formule III ou IV avec le sulfure d'hydrogène.

2. Procédé selon la revendication 1 dans lequel $R^3$ et $R^4$ représentent chacun l'hydrogène.

3. Procédé de préparation de composés de formule I selon la revendication 1, procédé selon lequel $R^1$ et $R^2$ représentent chacun, indépendamment l'un de l'autre, l'hydrogène ou un alkyle en $C_1$-$C_{12}$, $R^3$ et $R^4$ représentent chacun l'hydrogène, X représente un radical $-CR^5R^6-$, et $R^5$ et $R^6$ représentent chacun, indépendamment l'un de l'autre, l'hydrogène ou un méthyle.

4. Procédé de préparation de composés de formule I selon la revendication 1, procédé selon lequel $R^1$, $R^2$, $R^3$ et $R^4$ représentent chacun l'hydrogène, et X représente un radical $-CH_2-$, $-CH(CH_3)-$ ou $-C(CH_3)_2-$.

5. Procédé de préparation de composés de formule I selon la revendication 1, procédé selon lequel $R^1$ et $R^2$ représentent chacun un radical $-CH_2-CH(R^3)-CH_2-S-H$.

6. Procédé de préparation de composés de formule II selon la revendication 1, procédé selon lequel $R^3$ représente l'hydrogène, $R^8$ représente l'hydrogène ou un méthyle, $-CR^5R^6-$ représente un radical $-CH_2-$, $-CH(CH_3)-$ ou $-C(CH_3)_2-$, et n désigne un nombre entier de 1 à 4.

7. Compositions qui contiennent :

a) une résine époxydique dont la molécule renferme en moyenne plus d'un radical époxy, ou un pré-condensat durcissable, encore fusible et/ou soluble, de la résine époxydique,

b) au moins un composé de formule I et/ou II selon la revendication 1, et

c) éventuellement un accélérateur de durcissement.

8. Produits durcis qui peuvent être obtenus par chauffage de compositions selon la revendication 7.

9. Application des compositions selon la revendication 7 comme colles.